# EUROPEAN PATENT APPLICATION

(11) **EP 4 019 016 A1**
(43) Date of publication of application: **29.06.2022**
(21) Application number: 21171176.7
(22) Date of filing: 29.04.2021
(51) Int. Cl.: A61K 31/197, A61K 9/00, A61P 43/00

(54) **METHODS OF ADMINISTERING INTRAVENOUS BACLOFEN**

(30) Priority: 24.12.2020 US 202063130503 P
(71) Applicant: Allaysis, LLC, Marlton, NJ 08053 (US); Regents of the University of Minnesota, Minneapolis, Minnesota 55455-2020 (US)
(72) Inventor: Tata, Prasad, Windsor (US); Alhadeff, Dan Simon, Weston (US); Gomez, Adolfo L., Sicklerville (US); Schrogie, John, Landsdale (US); Kriel, Robert L., Minneapolis (US); Krach, Linda, Minneapolis (US); Cloyd, James C, Minneapolis (US); Diaz, Gilberto, Southwest Ranches (US); Coles, Lisa, Minneapolis (US); Schmitz, Natalie, Madison (US)
(74) Representative: Kramer Barske Schmidtchen Patentanwälte PartG mbB

(57) **Abstract**

Methods for the intravenous (IV) administration of a therapeutically effective amount of baclofen at a dosing regimen which mimics the oral pharmacokinetic profile of baclofen are disclosed. The disclosed methods meet bioequivalence criteria for both systemic exposure (AUC) and maximum plasma concentration (Cₘₐₓ) compared to oral administration.

## Description

### BACKGROUND

Baclofen (USP) is a skeletal muscle relaxant and anti-spastic agent. Baclofen is a structural analog of the inhibitory neurotransmitter gamma-aminobutyric acid (GABA) and acts as a GABA_{B} agonist at the level of the spinal cord. It is indicated for alleviation of signs and symptoms of spasticity, particularly for the relief of flexor spasms and concomitant pain, clonus, and muscular rigidity. Baclofen treatment can aid in restoring residual function following reversible spasticity. Intravenous (IV) baclofen may be used for short-term use in hospitalized patients whose long-term oral or intrathecal baclofen therapy has been temporarily interrupted, in order to maintain plasma levels of baclofen until the oral or intrathecal route is re-established. In April 2020, the FDA granted an orphan drug designation for IV baclofen for the prevention of baclofen withdrawal syndrome.

Baclofen is a white to off-white, odourless crystalline powder. It is slightly soluble in water (2090 mg/L), very slightly soluble in methanol and insoluble in chloroform. Baclofen's molecular formula is C₁₀H₁₂ClNO₂. Baclofen's molecular weight is 213.66 Da. Baclofen's chemical name is 4-amino-3-[4-chlorophenyl] butanoic acid (Baclofen Oral Tablets Package Insert, 2015). Baclofen's chemical structure is the following:

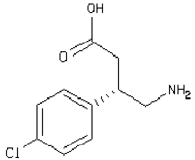

Baclofen is used to treat spasticity associated with a number of medical conditions, including cerebral palsy, multiple sclerosis, traumatic brain injury, spinal cord injury, stroke and neurodegenerative disorders. Baclofen is currently available as an oral tablet (10mg or 20mg tablets) and an intrathecal formulation (0.05 mg/mL, 0.5 mg/mL, or 2.0 mg/mL). The intrathecal formulation is used in conjuction with an implanted programmable pump to provide a constant infusion of the drug. Baclofen is typcially used on a long-term basis to maintain its therapeutic effect.

Baclofen is an important part of the therapeutic armamentarium for the treatment of spasticity present in patients afflicted with several neurological diseases. Administered chronically over an individualized dose range, it is well absorbed orally and is excreted largely unchanged by the kidneys. However, there are times when interruption of oral or intrathecal therapy is either necessary or unintended. Interruption of oral baclofen treatment may be caused by inability to maintain an oral route of administration due to intercurrent illness, surgery, non-compliance, or compromised gastrointestinal absorption. Examples of the latter include gastroenteritis, ileus, or post-operative ileus after prolonged anaesthesia. Interruption of intrathecal baclofen may occur when a programmable pump and/or catheter used for intrathecal administration needs to be removed, refilled, or replaced. Interruption of baclofen therapy is undesirable since it may lead to exacerbation of spasticity symptoms or, more seriously, an uncommon but severe withdrawal syndrome. This is a severe syndrome that can include:
- rebound increase in muscle tone and spasms
- hallucinations
- serotonin syndrome
- status epilepticus
- malignant hyperthermia
- elevated plasma creatine kinase, elevated transaminases, hepatic and renal failure, and in some cases, disseminated intravascular coagulation
- ultimately, withdrawal may also result in rhabdomyolysis, multisystem organ failure or death.

Baclofen exerts its anti-spasticity effect by binding to GABA_{B} receptors in the spinal cord. Prolonged baclofen exposure can reduce GABA_{B} binding density in rats. A reduction in GABA_{B} receptor sensitivity may explain the rapid rebound in CNS excitability of the central nervous system observed when baclofen is withdrawn. In summary, considering the known pharmacology of baclofen, maintenance or rapid replacement of baclofen levels is an essential therapeutic goal.

While oral and intrathecal baclofen are approved for the treatment of spasticity, there is no approved alternative dosage form to maintain therapy when these routes are not available. Patients experiencing interruption of baclofen therapy face a variety of clinical management problems including worsening of muscle tone, the side effects of other supplementary medications, the need for higher replacement doses when oral baclofen can be resumed, and the possibility of a difficult-to-manage baclofen withdrawal syndrome. When interruption of baclofen therapy occurs or can be predicted, the availability of a bridging therapy using intravenous baclofen would decrease the risk of these management problems. The challenges of treating the results of baclofen interruption demonstrate the need to utilize prophylactic measures when baclofen interruption occurs or is a possibility. Intravenous baclofen would provide clinicians with a dosage form that could be readily used in hospitalized patients to maintain or achieve a therapeutic response in a short period of time.

There is currently no drug therapy approved by the FDA for the prevention or treatment of baclofen withdrawal syndrome; however, warnings regarding baclofen withdrawal symptoms associated with abrupt discontinuation are included in the oral and intrathecal product labels. Pre-operative preparation at one specialty hospital for children with disabilities includes the tapering and discontinuation of oral baclofen prior to surgery in order to avoid baclofen withdrawal. This results in patients' muscle tone being inadequately managed with substitution of other, often more sedating medications with uncertain efficacy, on a short-term basis, as well as added stress and discomfort surrounding the perioperative experience. When oral baclofen therapy is interrupted, replacement with oral baclofen as soon as oral administration again becomes possible usually requires higher than typical doses. If symptoms of withdrawal persist, a benzodiazepine is administered as well. The diagnosis of baclofen withdrawal syndrome is often delayed due to the complex clinical presentation of the patients.

The current standard of care for baclofen withdrawal syndrome does not prevent serious sequelae, and in a few cases, has resulted in death. Therefore, it would be ideal to employ prevention strategies, such as intravenous baclofen, to avoid withdrawal symptoms which are associated with considerable morbidity and cost.

When interruption of baclofen therapy can be predicted, the availability of a bridging therapy, such as intravenous administration of baclofen, could decrease the risk of baclofen withdrawal. The challenges of treating baclofen withdrawal demonstrate the need to utilize prophylactic measures when baclofen withdrawal is a possibility. Baclofen IV would provide clinicians with a dosage form that could be readily used in the hospital and administered to patients to maintain a therapeutic response over a short period of time.

The primary hospital situations in which intravenous baclofen would be recommended in patients whose oral baclofen steady state plasma levels need be maintained include:
- Fasting (including oral medications) prior to surgery requiring general anesthesia due to risk of aspiration during surgery as well as the likelihood of post-surgical nausea and emesis;
- Patients with cerebral palsy and other physical disabilities are likely to undergo multiple surgeries, orthopedic and other, and represent a significant proportion of patients taking oral baclofen;
- Patients who are unconscious due to trauma, stroke, or other medical conditions rendering the patient unable to take medications orally;
- Difficulty swallowing after surgery due to an endotracheal tube; and
- Discontinuation of oral baclofen therapy with planned baclofen pump insertion surgery.

Further, interruption of intrathecal baclofen therapy may occur when the pump and/or catheter used in intrathecal administration needs to be removed, refilled, or replaced.

In such situations, baclofen therapy discontinuation may result in a rebound in spasticity symptoms and possible progression to a more severe withdrawal syndrome. Availability of an IV formulation of baclofen administered at doses intended to attain plasma concentrations comparable to those a patient has on oral or intrathecal maintenance therapy are expected to maintain the therapeutic effect of the oral or intrathecal formulation and reduce the likelihood of rebound or withdrawal until the oral or intrathecal route can be re-established.

At the present time, there is no established way to bridge a gap in oral or intrathecal therapy until oral or intrathecal dosing with baclofen can be re-instituted.

If left untreated, interruption of oral or intrathecal baclofen therapy can lead to baclofen withdrawal syndrome with no existing drug therapies approved or licensed to prevent or treat such withdrawal in the United States. Thus, availability of safe and effective therapeutic modalities to address this problem represents a critical unmet clinical need.

### BRIEF SUMMARY

Methods for the intravenous (IV) administration of a therapeutically effective amount of baclofen at a dosing regimen which mimics the oral pharmacokinetic profile of baclofen are disclosed. The disclosed methods meet bioequivalence criteria for both systemic exposure (AUC) and maximum plasma concentration (Cₘₐₓ) compared to oral administration.

In an embodiment, a method of administering baclofen to a subject comprises administering an intravenous baclofen infusion comprising about 75% to about 85% of a therapeutically effective amount of oral baclofen over a time period of about 180 to about 240 minutes.

In another embodiment, a method of administering baclofen to a subject comprises administering an intravenous baclofen infusion comprising about 80% of a therapeutically effective amount of oral baclofen over a time period of about 180 minutes.

The following are aspects of the invention:
1. A method of administering baclofen to a subject comprising:
   administering an intravenous baclofen infusion comprising about 75% to about 85% of a therapeutically effective amount of oral baclofen over a time period of about 180 to about 240 minutes.
2. The method of aspect 1, wherein the intravenous baclofen infusion further comprises a sterile solution of baclofen at a concentration of about 2mg/mL.
3. The method of aspect 1, wherein the intravenous baclofen infusion comprises about 80% of the therapeutically effective amount of oral baclofen.
4. The method of aspect 1, wherein the therapeutically effective amount of oral baclofen is about 20 mg.
5. The method of aspect 1, wherein the time period is about 180 minutes.
6. The method of aspect 1, wherein the administered intravenous baclofen infusion is bioequivalent to the therapeutically effective amount of oral baclofen based on systemic exposure as defined by AUC.
7. The method of aspect 1, wherein the administered intravenous baclofen infusion is bioequivalent to the therapeutically effective amount of oral baclofen based on maximum plasma concentration (Cₘₐₓ).
8. The method of aspect 1, wherein administering the intravenous baclofen infusion further comprises administering about 16 mg baclofen over a time period of about 180 minutes.
9. The method of aspect 1, wherein administering the intravenous baclofen infusion further comprises administering about 15 mg baclofen over a time period of about 180 minutes.
10. The method of aspect 1, wherein administering the intravenous baclofen infusion further comprises administering about 16 mg baclofen over a time period of about 240 minutes.
11. The method of aspect 1, further comprising administering the intravenous baclofen infusion when the subject is unable to take oral baclofen.
12. The method of aspect 11, further comprising administering the intravenous baclofen infusion temporarily until oral administration can be resumed.
13. The method of aspect 1, further comprising administering the intravenous baclofen infusion when the subject is unable to receive an intrathecal infusion of baclofen.
14. The method of aspect 13, further comprising administering the intravenous baclofen infusion temporarily until intrathecal administration can be resumed.
15. The method of aspect 1, further comprising administering the intravenous baclofen infusion chronically.
16. A method of administering baclofen to a subject comprising:
   administering an intravenous baclofen infusion comprising about 80% of a therapeutically effective amount of oral baclofen over a time period of about 180 minutes.
17. The method of aspect 16, wherein administering the intravenous baclofen infusion comprising about 80% of the therapeutically effective amount of oral baclofen further comprises administering about 16 mg baclofen.
18. The method of aspect 16, wherein the intravenous baclofen infusion further comprises a sterile solution of baclofen at a concentration of about 2mg/mL.
19. The method of aspect 16, wherein the administered intravenous baclofen infusion is bioequivalent to the therapeutically effective amount of oral baclofen based on systemic exposure as defined by AUC.
20. The method of aspect 16, wherein the administered intravenous baclofen infusion is bioequivalent to the therapeutically effective amount of oral baclofen based on maximum plasma concentration (Cₘₐₓ).
21. A method of administering an intravenous fluid comprising baclofen to a subject, the method comprising:
   administering the intravenous fluid to the subject over a specified time period to achieve a defined plasma baclofen delivery profile;
   wherein the defined plasma baclofen delivery profile comprises a maximum plasma baclofen concentration of about 250 to about 350 ng/mL.
22. The method of aspect 21, wherein the specified time period is about 180 to about 240 minutes.
23. The method of aspect 21, wherein the intravenous fluid comprises baclofen at a concentration of about 2 mg/mL.
24. The method of aspect 21, wherein the intravenous fluid comprises about 75% to about 85% of a therapeutically effective amount of oral baclofen.
25. The method of aspect 24, wherein the intravenous fluid comprises about 15 to about 16 mg baclofen.
26. The method of aspect 21, wherein administering the intravenous fluid further comprises administering about 16 mg baclofen over a time period of about 180 minutes.
27. The method of aspect 21, wherein administering the intravenous fluid further comprises administering about 15 mg baclofen over a time period of about 180 minutes.
28. The method of aspect 21, wherein administering the intravenous fluid further comprises administering about 16 mg baclofen over a time period of about 240 minutes.
29. The method of aspect 21, wherein the administered intravenous fluid is bioequivalent to the therapeutically effective amount of oral baclofen based on systemic exposure as defined by AUC.
30. The method of aspect 21, wherein the administered intravenous fluid is bioequivalent to the therapeutically effective amount of oral baclofen based on maximum plasma baclofen concentration (Cₘₐₓ).

These and other aspects and advantages of the present disclosure will become apparent from the subsequent detailed description and the appended claims. It is to be understood that one, some, or all of the properties of the various embodiments described herein may be combined to form other embodiments of the present invention.

Although methods and materials similar or equivalent to those described herein can be used in the practice or testing of the present disclosure, suitable methods and materials are described below. The materials, methods, and examples are illustrative only and not intended to be limiting. Other features of the disclosure are apparent from the following detailed description and the claims.

### BRIEF DESCRIPTION OF THE DRAWINGS

The following figures are included to illustrate certain aspects of the present invention and should not be viewed as exclusive embodiments. The subject matter disclosed is capable of considerable modifications, alterations, combinations, and equivalents in form and function, as will occur to those skilled in the art and having the benefit of this disclosure.
Fig. 1 illustrates plasma concentration-time profiles of baclofen after intravenous (5mg) and oral (10mg) administration.
Fig. 2 is a table showing mean pharmacokinetic parameters of baclofen following oral (10 mg) and intravenous (5 mg) administration.
Fig. 3 is a table showing mean pharmacokinetic parameters of baclofen following oral and intravenous administration in a dose escalation study.
Fig. 4 is a table showing dose escalation study population pharmacokinetic model estimates using Cohort 3.
Fig. 5 is a table showing dose escalation study population pharmacokinetic model estimates using Cohorts 2 and 3
Fig. 6 illustrates observed (DV) versus predicted plasma baclofen concentrations (Cohort 3 only, Ka = 0.8 hr⁻¹) (Ka = rate constant).
Fig. 7 illustrates observed (DV) versus predicted plasma baclofen concentrations (Cohort 3 only, Ka = 0.6 hr⁻¹).
Fig. 8 illustrates observed (DV) versus predicted plasma baclofen concentrations (Cohorts 2 and 3, Ka = 0.8 hr⁻¹).
Fig. 9 illustrates observed (DV) versus predicted plasma baclofen concentrations (Cohorts 2 and 3, Ka = 0.6 hr⁻¹).
Fig. 10 is a table summarizing bioequivalence results for various infusion lengths using population models developed using Cohort 3 only.
Fig. 11 is a table summarizing bioequivalence results for various infusion lengths using population models developed using Cohorts 2 and 3.
Fig. 12 is a table showing individual pharmacokinetic model estimates using Cohort 3.
Fig. 13 illustrates individual pharmacokinetic model fits.
Fig. 14 illustrates 15 mg IV baclofen average concentration-time profiles.
Fig. 15 illustrates 16 mg IV baclofen average concentration-time profiles.
Fig. 16 is a table showing mean pharmacokinetic parameter estimates using non-compartmental analysis.
Fig. 17 is a table showing predicted bioequivalence for various infusion lengths when each subject of Cohort 3 was modeled individually (dose = 15 mg).
Fig. 18 is a table showing predicted bioequivalence for various infusion lengths when each subject of Cohort 3 was modeled individually (dose = 16 mg).
Fig. 19 illustrates simulated plasma baclofen concentrations after does of 20mg PO, 15 mg IV infused over 180 minutes, and 16 mg IV infused over 180 and 240 minutes.
Fig. 20 is a table showing baclofen bioequivalence simulations for various infusion regimens assuming 80% bioavailability (N=30).
Fig. 21 illustrates mean plasma baclofen concentration-time profiles after baclofen IV infusion of 16 mg over 180 minutes (2 mg/mL) (Treatment B) and single oral administration of 20 mg baclofen tablet (Treatment D).
Fig. 22 is a table summarizing mean plasma pharmacokinetic parameters of baclofen by treatment (A, B, C, and D).
Fig. 23 is a table showing a statistical analysis of the natural log-transformed systemic exposure of baclofen.

### DETAILED DESCRIPTION

### Terms and Definitions

Unless explained otherwise, all technical and scientific terms used herein have the same meaning as commonly understood to one of ordinary skill in the art to which this disclosure belongs. All references herein are incorporated by reference. The following explanations of terms and abbreviations are provided to better describe the present disclosure and to guide those of ordinary skill in the art in the practice of the present disclosure.

To provide a more concise description, some of the quantitative expressions given herein are not qualified with the term "about." It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

The term "subject" as used herein, refers to an animal, preferably a mammal, most preferably a human, who has been the object of treatment, observation or experiment.

The term "therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of at least one of the symptoms of the disease or disorder being treated and/or reduction in the frequency and/or severity of at least one of the symptoms of the disease or disorder being treated.

### Clinical Pharmacology

The present disclosure provides various methods for the intravenous administration of a therapeutically effective amount of baclofen at a dosing regimen which mimics the oral pharmacokinetic profile of a given baclofen dose. The disclosed methods meet bioequivalence criteria for both systemic exposure (AUC) and maximum plasma concentration (Cₘₐₓ) compared to oral administration. The disclosed methods are intended for the use of a sterile baclofen IV injection (e.g., 2 mg/mL) drug product.

Two pilot studies were conducted to characterize baclofen pharmacokinetics and safety following intravenous administration and to access absolute bioavailability of IV and oral baclofen. These studies are described in: A Pilot Study Assessing Pharmacokinetics and Tolerability of Oral and Intravenous Baclofen in Healthy Adult Volunteers, Suresh K. Agarwal, Robert L. Kriel, James C. Cloyd, Lisa D. Coles, Lisa A. Scherkenbach, Michael H. Tobin, and Linda E. Krach, Journal of Child Neurology 2014, 30(1) 37-41; and A Randomized Dose Escalation Study of Intravenous Baclofen in Healthy Volunteers: Clinical Tolerance and Pharmacokinetics, Schmitz NS, Krach LE, Coles LD, Mishra U, Agarwal SK, Cloyd JC, Kriel RL. PM&R, 2017, Volume 9(8), 743-750, both hereby incorporated by reference as though fully set forth herein. Based on the results of these two prospective pharmacokinetic studies, which included 10- to 60-minute bolus infusions of IV baclofen, the estimated mean bioavailability of oral baclofen was approximately 74-85%, or approximately 80%. While these initial studies demonstrated comparability of AUCs (AUC = area under the concentration-time curve) following oral and IV baclofen, they showed that the Cmax (Cmax = maximum plasma concentration) after bolus IV baclofen infusion exceeded that following oral dosing. In order to meet bioequivalency criteria for both exposure (AUC) and peak concentration (Cₘₐₓ), it was concluded that infusion times needed to be longer. To that end, modeling and simulation studies were performed to predict IV baclofen dosing regimens that would be bioequivalent to a 20 mg oral dose with respect to both AUC and Cₘₐₓ. These simulations formed the basis for a pivotal bioequivalence study where three different dosing regimens of baclofen IV (15 mg infused over 3 hours; 16 mg infused over 3 hours; and 16 mg infused over 4 hours, respectively) were compared to a single dose of a 20 mg oral tablet. Each of these IV regimens was bioequivalent to the 20 mg oral dosing but the 16mg/3 hours dosing best met the bioequivalence criteria.

Results of the two pilot studies referenced above are briefly described as follows:

### Study 1: Two-Way Crossover Study of Oral and Intravenous Baclofen in Healthy Adult Volunteers

The objective of the study was to characterize baclofen pharmacokinetics following oral and IV administration following low doses in healthy adult volunteers and determine the safety profile of an IV baclofen formulation.

This study was a randomized, open label, balanced, two-treatment, single dose, crossover design in normal, healthy adult human subjects under fasting conditions.

Initially, three adult volunteers received a single 3 mg IV and a 5 mg oral baclofen dose on separate study days. On the first day of drug administration in the study, one subject was given baclofen intravenously, and two subjects were given baclofen orally. On the second day of drug administration in the study, those that received baclofen orally received the IV dose and the individual who received the IV dose received the oral dose. Clinical tolerance was assessed, and plasma concentrations of baclofen were measured from the first three subjects before additional subjects were entered into the study. Nine additional adult volunteers received single 5 mg IV and 10 mg oral baclofen doses on separate days. Randomization occurred in a 1:1 ratio, such that all subjects received IV and oral baclofen on separate study days, but not necessarily in the same order. A total of twelve (12) subjects completed the clinical phase of the study. Plasma samples of 12 subjects were analyzed. However, the data from nine (9) subjects who received 5 mg IV and 10 mg oral tablet were considered for pharmacokinetic analysis and the calculation of within-subject variability. The three subjects who received 3mg IV/5mg oral baclofen were excluded from the pharmacokinetic analysis because more than half of the plasma concentrations were below the limit of quantitation (BLQ). Therefore, meaningful estimation of AUC was not possible for these subjects.

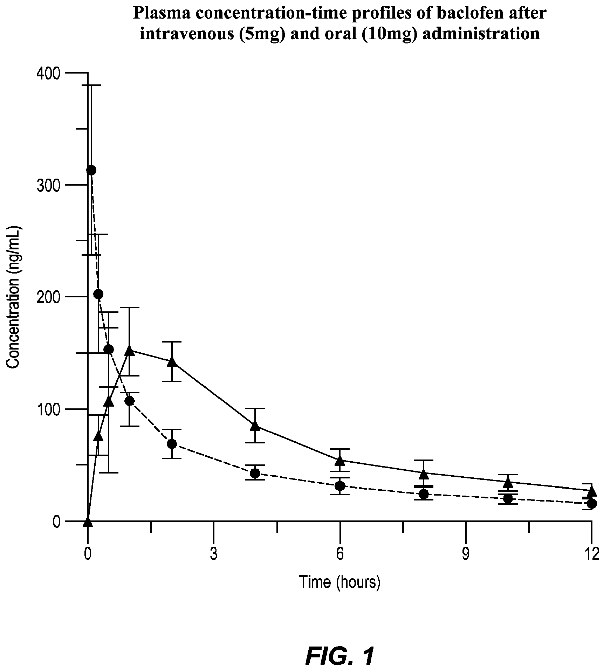

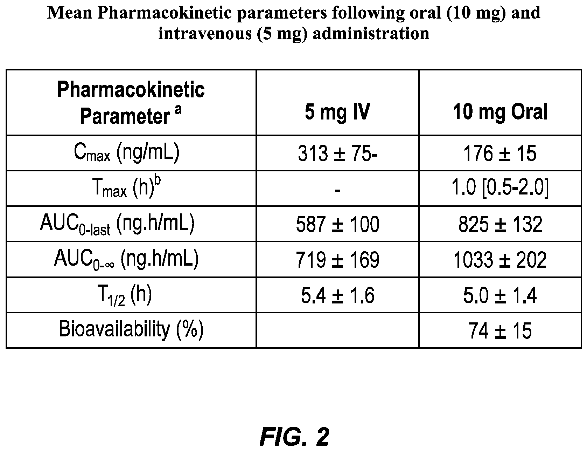

Mean plasma concentration profile and mean pharmacokinetic parameters are presented in Fig. 1 and Fig. 2, respectively.

Based on the results presented above (small sample size, n=9), the estimated mean absolute bioavailability of oral baclofen was 74%, indicating that approximately 25% of a 10-mg dose is either not absorbed or undergoes first-pass metabolism prior to drug reaching systemic circulation. The IV dose, infused over 15 minutes, produced a similar AUC; although Cₘₐₓ was higher and Tₘₐₓ (time to maximum plasma concentration) earlier than with the oral dose. It also implies that a lower intravenous baclofen dose will be needed when substituted for oral doses. The between-subject variability in exposure (area under the curve) was similar in both intravenous and oral arms (coefficient of variation: 18%-24%). Both 3 and 5mg IV baclofen were well tolerated. The results indicated that additional studies with a larger sample size are needed to precisely determine the population's mean absolute bioavailability and adverse event profile.

### Study 2: Dose Escalation Study of IV Baclofen in Healthy Adult Volunteers

Following completion of the first-in-human study, a dose escalation study of IV and oral baclofen in 36 healthy volunteers was carried out using a randomized crossover oral/IV dose design and incorporating the investigational baclofen IV injection. The objectives of the study were: 1) characterize the pharmacokinetics of clinically relevant doses of oral and intravenous baclofen, 2) determine the safety profile of oral and intravenous baclofen, and 3) estimate bioequivalence parameters (AUC and Cₘₐₓ) for 10- and 60-minute (only 15 mg dose) infusions of baclofen.

The general design of the study involved three cohorts of 12 subjects. Initially 3 subjects were randomized to receive the lowest dosing regimen. These subjects received a 7.5 mg IV infusion, followed by a washout period of 2 or more days, followed by a 10 mg oral dose. Upon demonstration that these 3 subjects tolerated the IV dose, the remaining 9 subjects were randomized for dosing. Twelve subjects completed Cohort 1 and received a 7.5 mg IV and a 10 mg oral dose. The IV baclofen doses were based on a 75% bioavailability observed in the pilot study. In Cohort 2, 12 different subjects received 11.5 mg of IV and 15 mg of oral baclofen. In Cohort 3, another 12 subjects received 20 mg of oral and 15 mg of IV baclofen. Once all subjects had completed the 15 mg IV dose without complications, an additional dosing period was added to this Cohort in which 15 mg baclofen was infused over 60 min to 12 subjects. Subjects were observed at a clinical research facility for 24 hours following all doses and were assessed for nystagmus, ataxia and sedation (using the Modified Sanford Sleepiness Scale). Blood samples were collected between 0 - 24 hours. A validated, liquid chromatography-mass spectroscopy assay was used to measure plasma baclofen concentrations.

Pharmacokinetic analysis was performed with Phoenix (Centara), a pharmacokinetic data analysis package. Pharmacokinetic parameters, including the observed maximum concentration (Cₘₐₓ), the observed time to maximum concentration (Tₘₐₓ), area under the concentration-time curve (AUC), clearance (CL), and volume of distribution (V_{d}), were estimated using non-compartmental analysis. The absolute oral bioavailability was calculated by dividing the dose-normalized AUC after oral dosing by the dose-normalized AUC after intravenous dosing. For an exploratory study of bioequivalence, the 90% confidence intervals of the geometric mean AUC(0-t), AUC(0-∞) and Cₘₐₓ oral/IV ratios were determined using the 60-minute infusion as the test dose and the oral 15 mg dose as the reference.

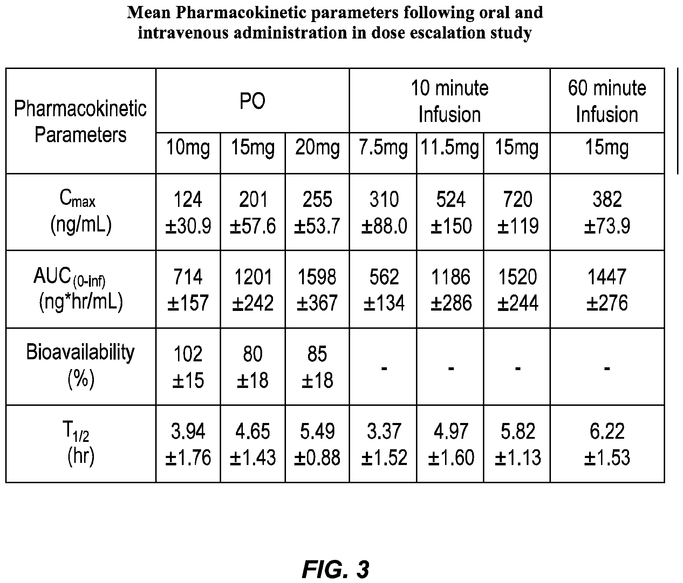

The estimated mean bioavailability for all cohorts was 89%. However, because there were limited baclofen concentrations for cohort 1 above the limit of quantification, the bioavailability estimates for cohorts 2 and 3 (85.8 and 81.2, respectively) are thought to be more reflective of the actual value. When the mean bioavailability for these groups were considered, an IV dose 15-20% smaller than oral doses would likely produce equivalent total systemic exposure. Mean Pharmacokinetic parameters are summarized in Fig. 3.

Bioequivalence of baclofen IV and oral baclofen was demonstrated when using total systemic exposure, AUC₀₋ₜ and AUC_{0-inf} at 10-min infusions at all dose levels but not for 15 mg at 60-min infusion. However, the mean Cₘₐₓ ratios of 10- and 60-minute IV infusions to oral baclofen were 263% and 149% with confidence intervals (CIs) of 247-281% and 135-165%, respectively, and are not bioequivalent.

Safety Results: Mild, self-limited adverse effects were seen following both oral and IV baclofen doses. Adverse events associated with known pharmacological effects of baclofen included nystagmus, ataxia, and sedation. With regard to sedation, all subjects, regardless of route of administration or dose, were awake except for one individual who took 20 seconds to awaken three and a half hours after a 10mg oral dose.

All subjects could walk without assistance. One subject experienced mild transient ataxia after the oral administration of 20 mg baclofen. Of the 12 subjects in each dosing arm, 2, 6, and 6 subjects experienced asymptomatic nystagmus following 10-minute infusions of 7.5mg, 11.5mg, and 15mg, respectively. Two subjects experienced mild transient ataxia and two subjects had nystagmus after the 15mg 60-minute infusion. Evidence from the two prospective studies in healthy volunteers indicate that adverse effects after single intravenous infusions of clinically relevant baclofen doses are generally mild and that the ratio of an IV to an oral dose is approximately 80-85%.

Conclusion: Based on the results from the two prospective pharmacokinetic studies in healthy volunteers, it can be concluded that adverse effects after single intravenous infusions of clinically relevant baclofen doses are generally mild and that the ratio of an IV to an oral dose is approximately 80-85%, with exposures meeting the bioequivalence criteria (AUC) but not the peak concentration criteria (Cₘₐₓ).

In order to meet bioequivalence criteria for both exposure and peak concentration, infusion time needs to be longer. To that end, modeling and simulation studies were performed to select appropriate infusion rates and identify a dose regimen which would best mimic the oral pharmacokinetic (PK) profile.

To facilitate a better understanding of the present disclosure, the following examples of preferred or representative embodiments are given. In no way should the following examples be read to limit, or to define, the scope of the invention. Numerous embodiments within the scope of the claims will be apparent to those of ordinary skill in the art; thus, the following non-limiting examples only describe particular embodiments of the disclosure.

### EXAMPLE 1 - MODELING AND SIMULATION STUDY

### Population Modeling Approach

### Model Building

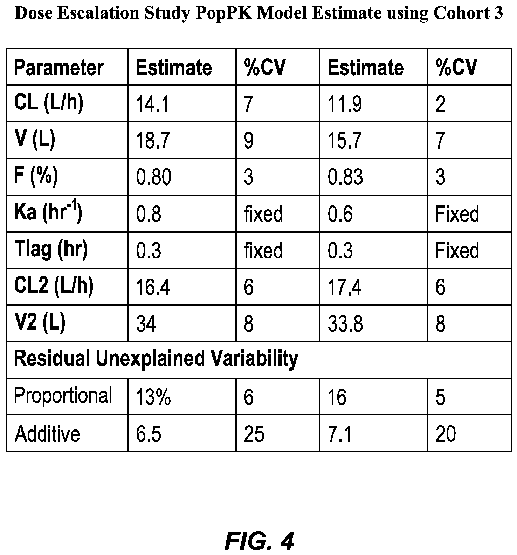

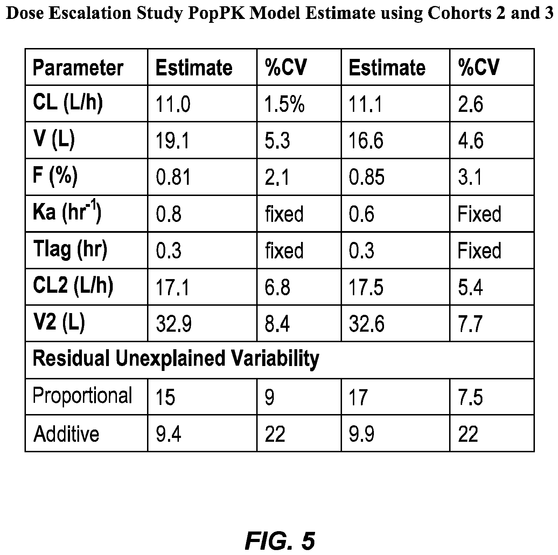

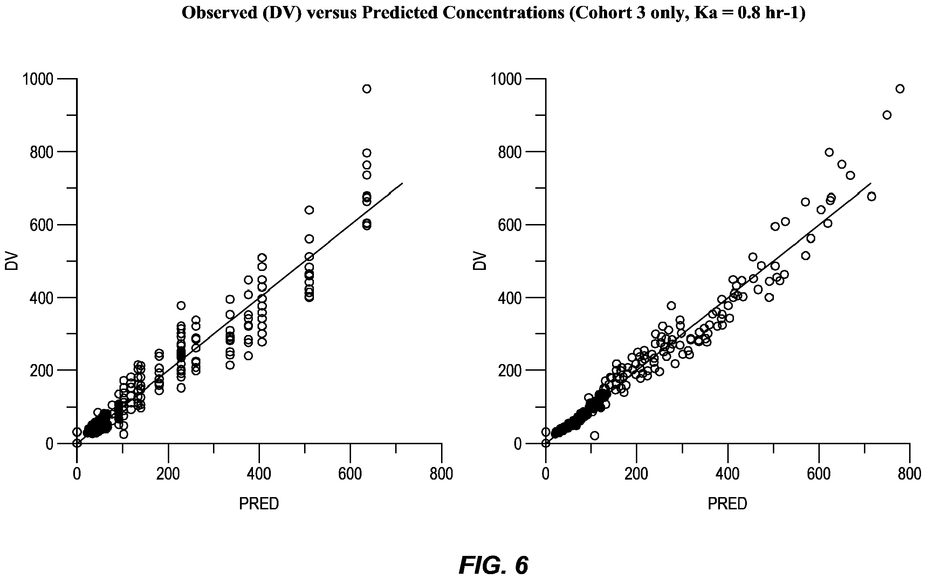

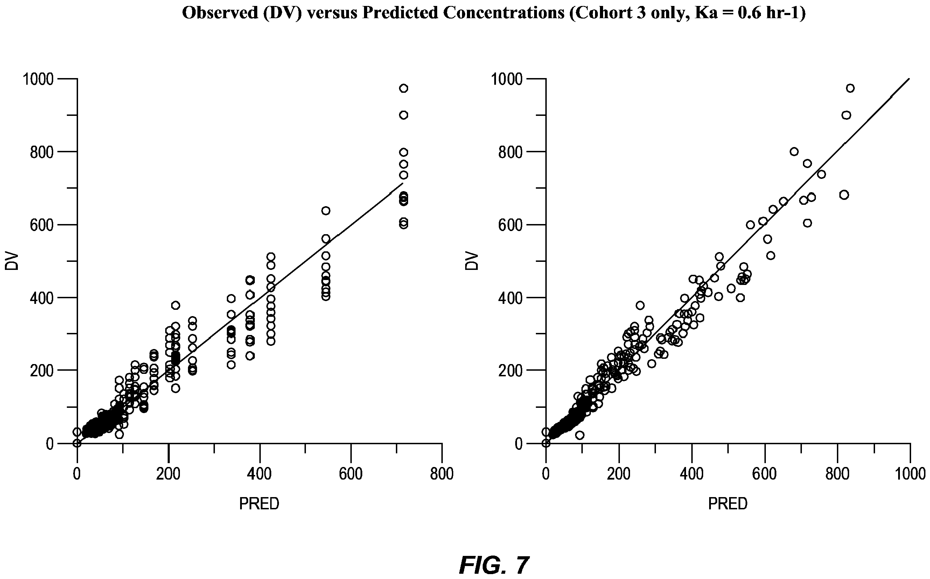

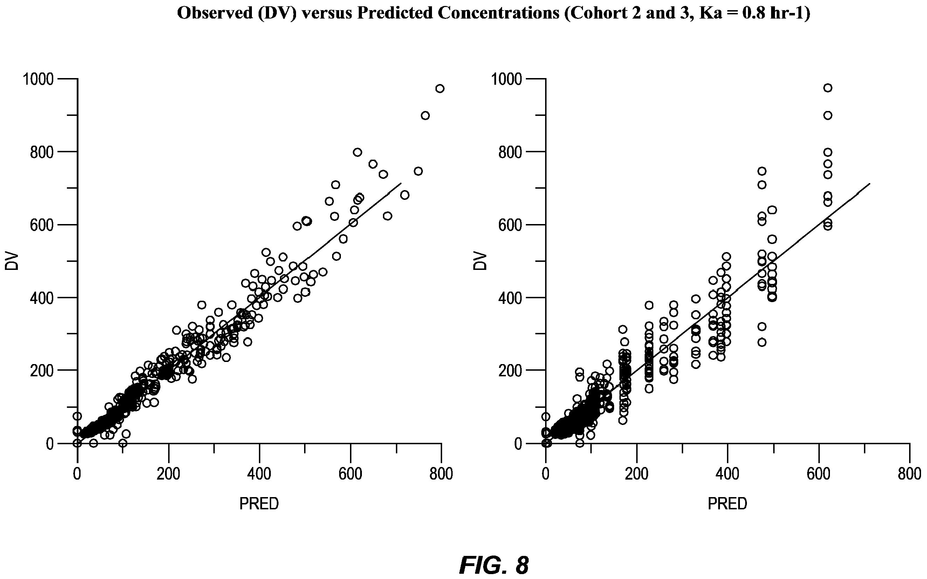

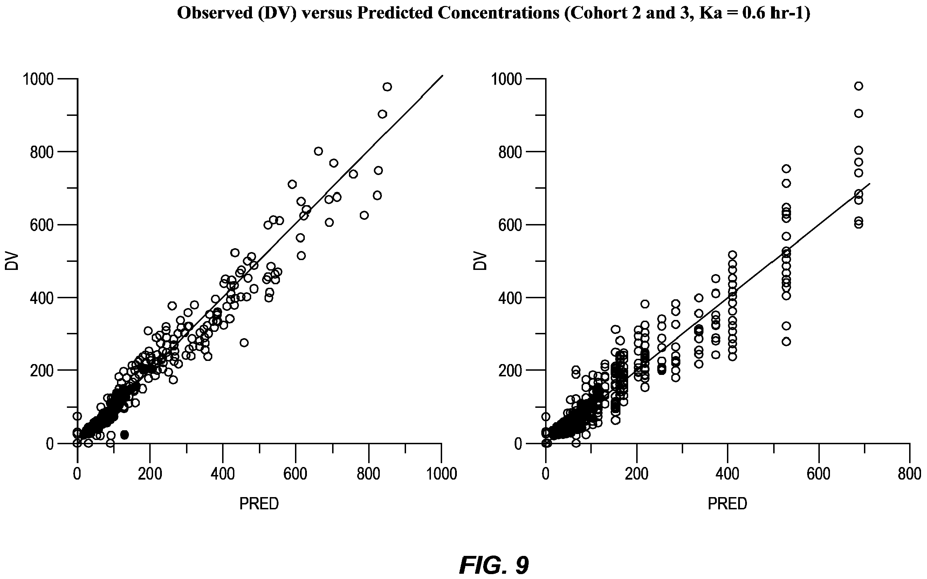

Plasma concentrations from cohorts 2 and 3 from Study 2 described above were used to build a two-compartment model simultaneously describing the IV and PO routes of administration. Population PK models were developed using data from cohort 3 and cohorts 2 and 3 combined (see Figs. 4 and 5). It is worth noting that the PK parameter estimates from both study populations were similar. Two PK models with fixed absorption rate constants (Ka) of 0.6 and 0.8 hr⁻¹ were used. These were determined from estimates from the population pharmacokinetic model and literature. Visual predictive checks for these models are shown in Figs. 6-9.

### Simulations

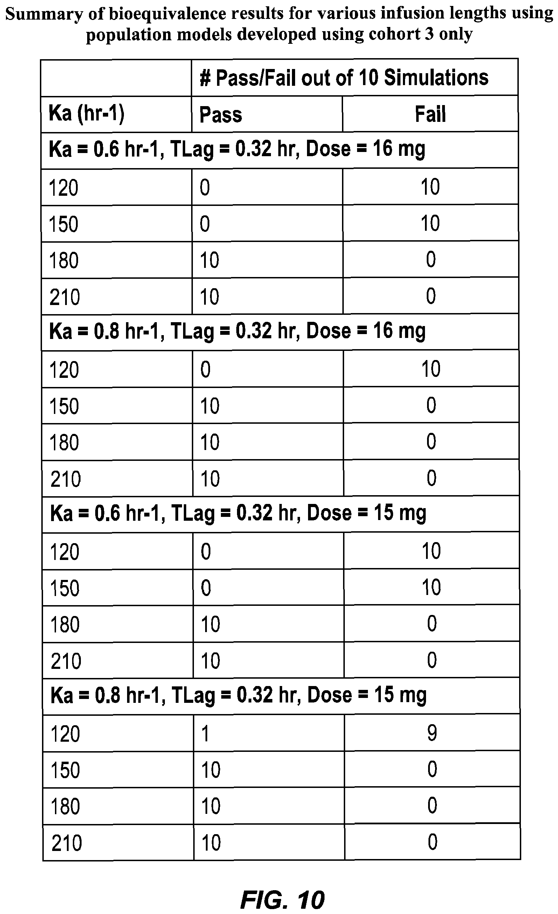

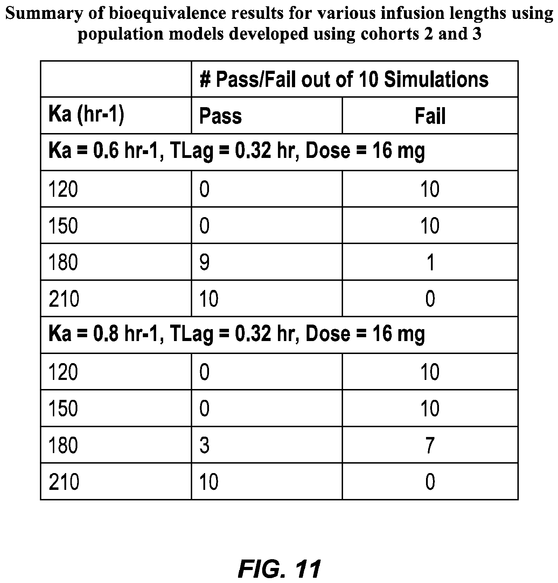

To assess the effect of infusion duration on Cₘₐₓ and AUC, simulations were performed using the population PK parameter estimates determined from the baclofen dose ranging study. Using the models described above, simulations (n=10) of 30 subjects were completed using a 20 mg oral dose and IV doses of 15 and 16 mg infused over 120, 150, 180, and 210 minutes. The simulated data were then analyzed using non-compartmental analysis and bioequivalence of the maximum concentration and area under the curve was assessed. Figs. 10 and 11 summarize how many times of the 10 simulations that the infusion rate met bioequivalence when 16 and 15 mg doses were used, respectively.

### Individual Modeling Approach

### Model Building

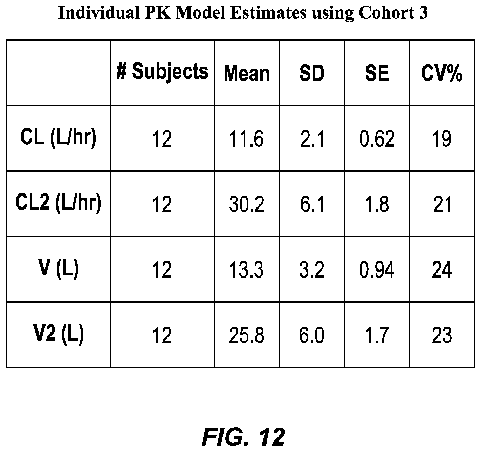

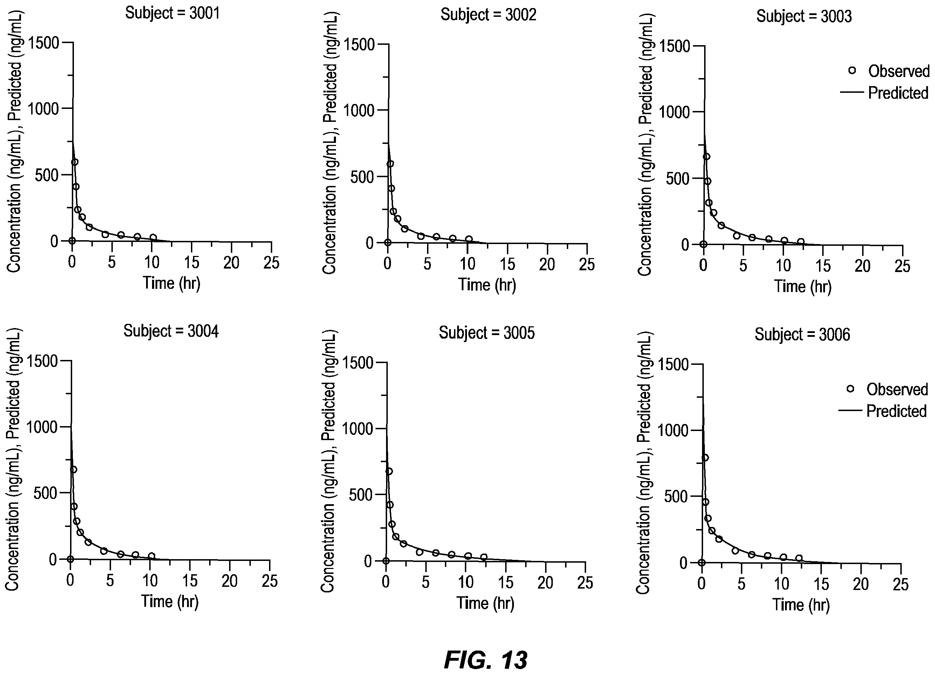

As an alternative approach, individual PK models were developed using the plasma concentrations from each subject in cohort 3 with IV dosing as described in population modeling approach. It is worth noting that the PK parameter estimates (Fig. 12) were similar to those from the population models (Figs. 4 and 5). A goodness of fit plot is shown in Fig. 13.

### Simulations

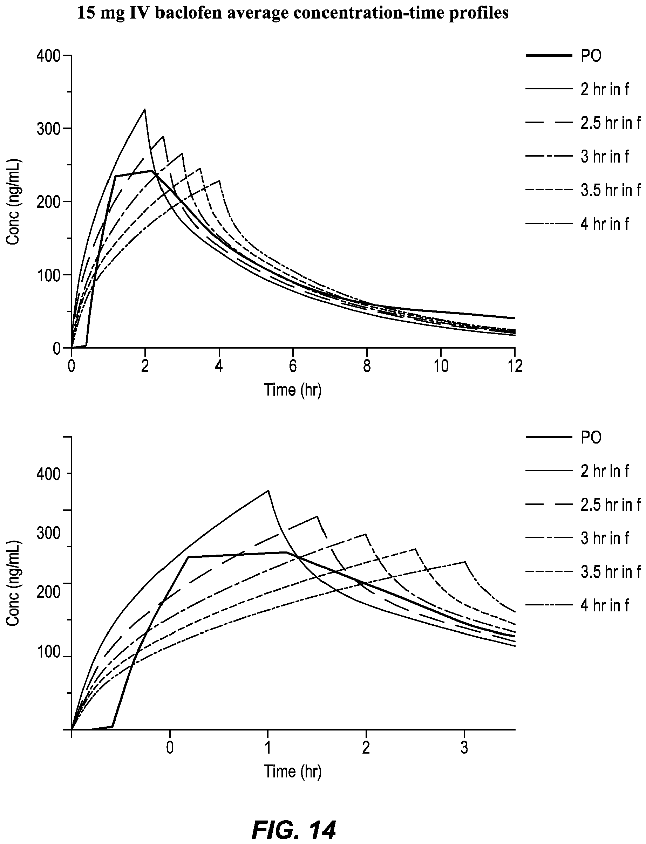

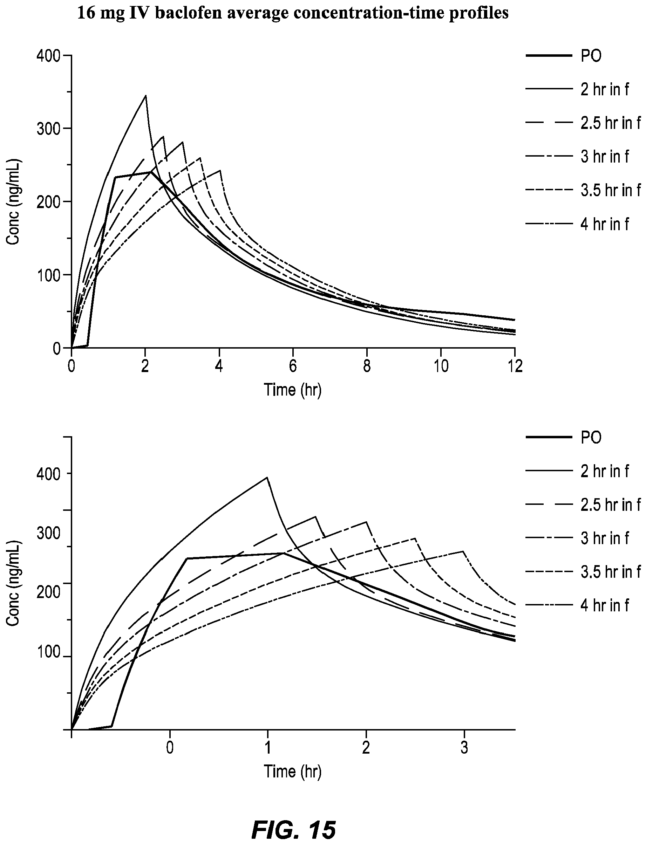

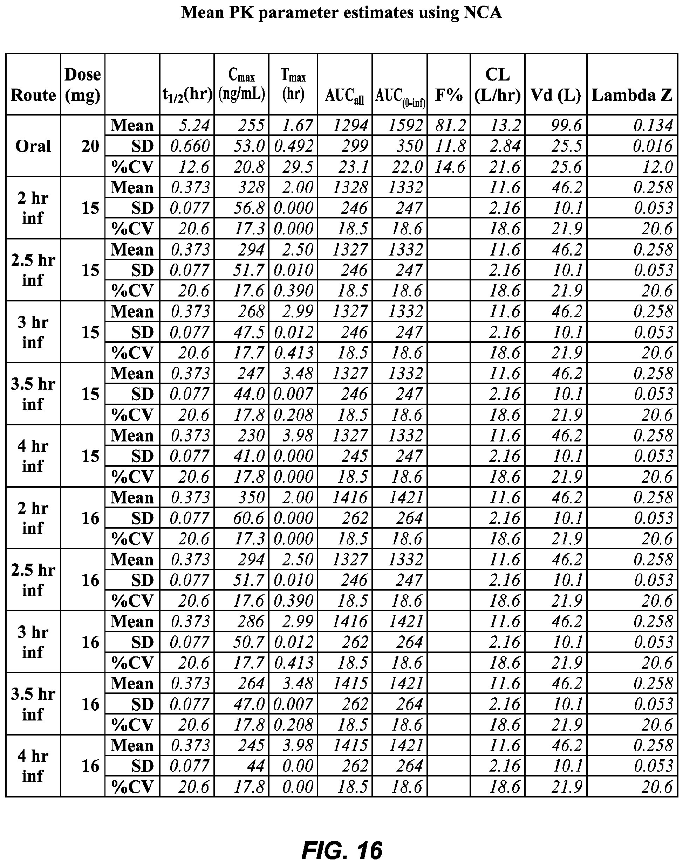

Using the models described above, various infusion durations were simulated for each subject using 15 and 16 mg doses. The simulated IV data were then analyzed using non-compartmental analysis and bioequivalence of the maximum concentration and area under the curve was assessed comparing the exposure parameters for IV dosing with those calculated for oral dosing. Average concentration-time profiles are shown in Figs. 14 and 15. The results of the bioequivalence testing are summarized in Fig. 16.

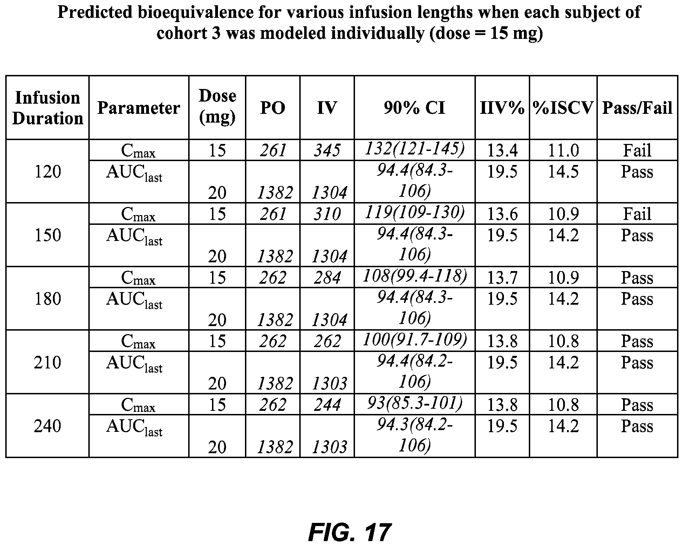

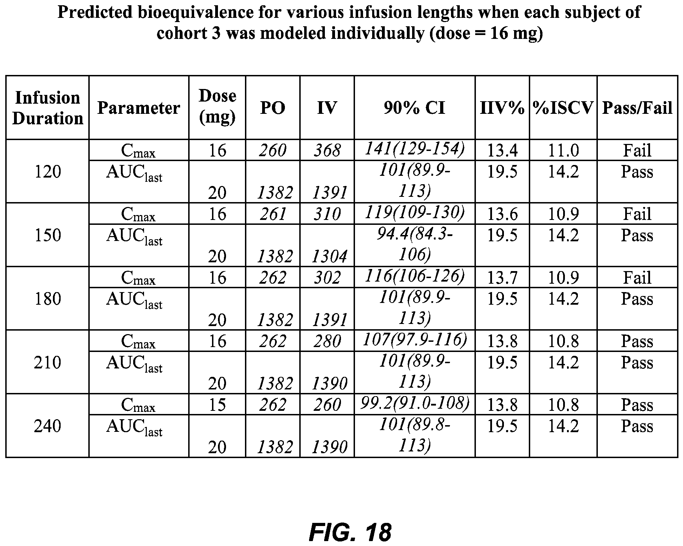

Based on the above population and individual modeling approach, simulations, and applying these results to a virtual crossover study (see Figs. 17 and 18), a pivotal bioequivalence study was designed to assess baclofen IV injection at 15 mg and 16 mg dosage strength and a regimen consisting of infusion over 180 or 240 minutes.

### Rationale for Pivotal Bioequivalence Study

An important goal for adjusting the dose and duration of a baclofen infusion was to achieve bioequivalent systemic exposure (Cₘₐₓ and AUC) between a single-dose baclofen IV infusion and a 20 mg oral baclofen tablet. When considering the two initial pharmacokinetic analyses of the first in human and dose escalation studies of IV baclofen, the mean bioavailability of oral baclofen following a single dose in 45 subjects was approximately 86% with a wide inter-patient variability (%F ranging from 42-132%). When clinically relevant doses (11.5 and 15 mg) were selected, the mean oral bioavailability was 80 and 85% respectively.

For the design of this study, IV doses of 75 and 80% (15 and 16 mg) of the standard oral dose (20 mg) were chosen, a conclusion which was consistent with the results of the previous first in human study. In addition, results of the 10- and 60-minute infusions suggested that further prolongation of the infusion time would achieve the desired comparability of Cₘₐₓ between the IV and oral doses (see below).

### Conclusion: Modeling and Simulation to Assess the Effect of Infusion Duration on Cₘₐₓ and AUC Following Administration of Intravenous Baclofen

The results of the dose-ranging study revealed that baclofen bioavailability is variable among patients and may be dependent on dose. Systemic exposures after IV administration of baclofen will not vary as will oral administration, since bioavailability is excluded as a source of variability following IV administration. Dose adjustments in patients receiving IV baclofen replacement therapy protect against either low plasma concentrations possibly leading to an increased risk in spasticity or high concentrations leading to possible CNS-related adverse effects.

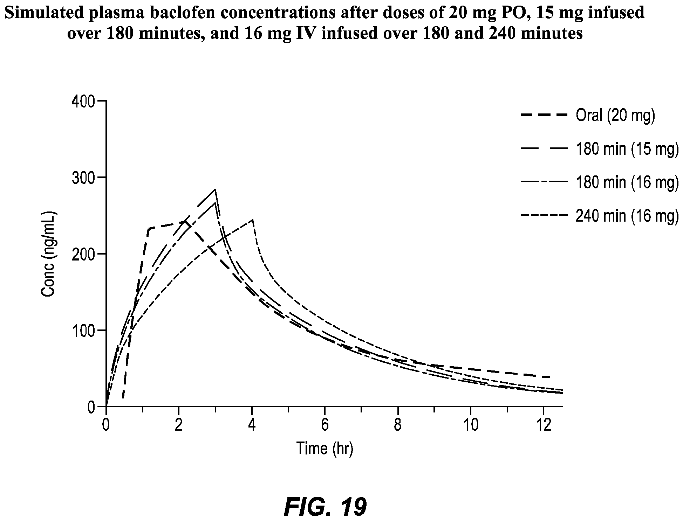

To assess the effect of infusion duration on Cₘₐₓ, a population pharmacokinetic model was developed using the observed plasma concentrations of baclofen following oral dosing of 20 mg and IV dosing of 15 mg infused over 10 and 60 minutes. Plasma concentrations of baclofen were best described by a two-compartment model, displaying a very rapid tissue distribution phase indicative of highly perfused tissues such as the liver, lung and brain and a more prolonged elimination phase. The modeling results revealed that the mean distribution t_{1/2} was rapid (∼0.3 hr), indicating that elevated plasma levels of baclofen would be short-lived once an infusion was stopped. Population mean terminal t_{1/2} (β) is approximately 4.2 hours, which is comparable to the values based on non-compartmental analysis. A mean bioavailability value of 84% was estimated. Using the PK model, baclofen concentrations for a 20 mg oral dose, a 15mg IV dose infused over 180 minutes, and 16 mg IV dose infusions administered over 180 and 240 min were simulated. This allowed a determination of the effect of infusion duration on baclofen Cₘₐₓ. The simulation results are summarized in Fig. 19.

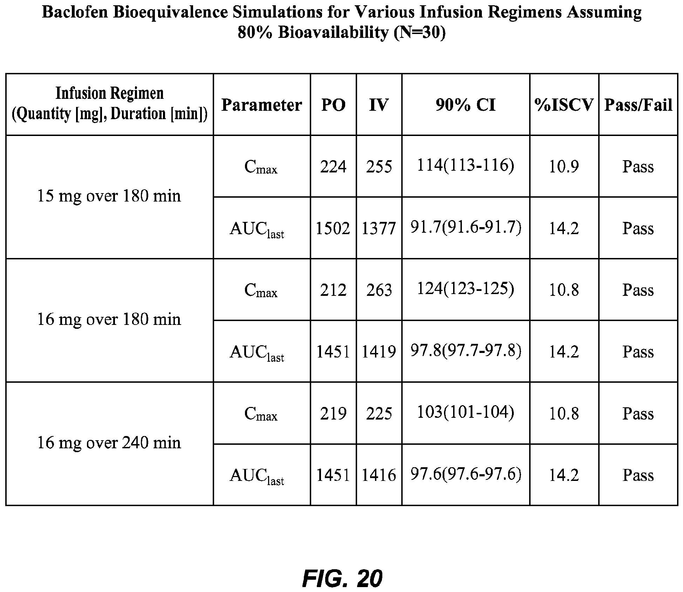

When virtual bioequivalence testing was performed with the simulated data, as shown in Fig. 20, it appeared that infusion rates of 180 and 240 minutes would result in similar Cₘₐₓ and AUC. These results provided support for the selected infusion durations of 180 and 240 minutes that were tested in this clinical trial.

### EXAMPLE 2 - CLINICAL BIOEQUIVALENCE STUDY

### Overall Study Design and Plan

This study was designed as a two-part, single-dose bioequivalence study of baclofen injection (test formulation) compared to commercial baclofen tablets (reference product) in healthy adult volunteers under fasted conditions. Part 1 of this study was a single-dose, open-label, four-treatment, randomized, crossover bioequivalence study of baclofen tablets and baclofen injection in healthy adults under fasted conditions. If needed, Part 2 of this study was to be a single-dose, two-way crossover between the most promising infusion of baclofen injection from Part 1 and baclofen tablets. Because the study objective of bioequivalence between baclofen infusions(s) and baclofen tablets was demonstrated in Part 1 of the study, planned Part 2 was not conducted.

Subjects were randomized to receive each of 4 treatments in a randomized, crossover fashion:
- 15 mg of baclofen injection administered intravenously over 180 minutes
- 16 mg of baclofen injection administered intravenously over 180 minutes
- 16 mg of baclofen injection administered intravenously over 240 minutes
- 20 mg of baclofen tablet administered orally

### Primary Objective

Identify the dose of baclofen injection (amount and rate of infusion) in healthy subjects that would provide comparable systemic exposure with a single dose of 20 mg baclofen tablet as evidenced by bioequivalence.

### Secondary Objectives

Determine the single dose pharmacokinetics and absolute bioavailability of 20 mg baclofen tablets versus 15 mg and 16 mg of baclofen injection infused at different rates in healthy adults.

Evaluate the safety and tolerability of baclofen injection given as an infusion and as baclofen tablets given orally.

### Discussion of Study Design, Including the Choice of Control Groups

This study design was a single-dose, open-label, four-treatment, randomized, crossover. Using this design, each subject was to serve as his/her own control, precluding the need for a separate control group. The study design is typical for bioavailability and bioequivalence studies.

Each treatment administration was separated by a washout period of at least 3 days. Each dose was administered following a 10-hour overnight fast.

During each study period, 6 mL blood samples were obtained prior to study drug administration and at selected times through 48 hours after drug administration. In the study periods in which subjects received an IV infusion, PK sampling was timed relative to the start of the infusion. Plasma pharmacokinetic samples were analyzed for baclofen using a validated achiral bioanalytical method. Appropriate pharmacokinetic parameters were calculated for each formulation using non compartmental methods.

Blood and urine were collected for clinical laboratory testing at screening and at end-of-study/early termination. Urine was collected for drug, alcohol, and cotinine testing at screening and check-in. Female subjects had urine collected for pregnancy testing at screening, check-in, and at the end of the study/early termination.

Thirty-two subjects were planned for Part 1. Two subjects who withdrew from the study were replaced.

### Treatments

Subjects were administered a single dose of each treatment in a randomized, sequenced fashion. Each dose of baclofen injection was given via IV infusion. The dose of baclofen tablet was given orally with 240 mL (8 fl oz) of room temperature water.

### Identity of Investigational Products

### Test Product: Baclofen Injection 2 mg/mL

Treatments A, B, and C:
- Treatment A dose = 15 mg of baclofen injection administered intravenously over 180 minutes
- Treatment B dose = 16 mg of baclofen injection administered intravenously over 180 minutes
- Treatment C dose = 16 mg of baclofen injection administered intravenously over 240 minutes

Control Product: Treatment D:
- Baclofen Tablet, 20 mg , dose = 1 x 20 mg tablet

All safety and pharmacokinetic measurements obtained in this study are standard and widely used for bioavailability and bioequivalence studies.

Blood samples were obtained to determine the pharmacokinetic profile and exposure of baclofen after each treatment.

Concentration-time data for baclofen were analyzed using noncompartmental methods in Phoenix^{™} WinNonlin^{®} (Version 6.3, Certara L.P.). During the pharmacokinetic analysis, concentrations below the limit of quantitation (BLQ) up to the time of the first quantifiable concentration were treated as zero. Embedded (values between 2 quantifiable concentrations) and/or terminal BLQ concentrations were treated as "missing". Actual sample times, relative to the time of dose, were used in the pharmacokinetic analysis.

The following pharmacokinetic parameters were determined:

| | |
|---|---|
| Cₘₐₓ | Maximum plasma concentration, determined directly from individual concentration-time data |
| Tₘₐₓ | Time of the maximum plasma concentrations |
| λ_{z} | The observed terminal rate constant; estimated by linear regression through at least three data points in the terminal phase of the log concentration-time profile |
| T_{1/2} | The observed terminal half-life, calculated as: T_{1/2}= ln(2)/ λ_{z} |
| AUCₗₐₛₜ | Area under the plasma concentration-time curves from time-zero to the time of the last quantifiable concentration; calculated using the linear trapezoidal rule |
| AUC_{inf} | Area under the plasma concentration-time curves from time-zero extrapolated to infinity; calculated as: AUC_{inf} = AUCₗₐₛₜ + Cₗₐₛₜ/λ_{z} |
| AUC_{Extrap} (%) | The percentage of AUC_{inf} based on extrapolation |
| Cₗₐₛₜ | The last plasma concentration determined directly from individual concentration-time data |
| Tₗₐₛₜ | Time of the last quantifiable plasma concentration |
| CL_{T} | Total systemic clearance, calculated after IV dosing, calculated as: CL_{T}=Dose/AUC_{inf} |
| Vz | Volume of distribution after IV dosing, calculated as: Vz = Dose/AUC_{inf} x λz |

Blood (plasma) pharmacokinetic characteristics were assessed after each dose of investigational product.

During each baclofen injection treatment period, samples were drawn at 0 hour (predose) and 0.5, 1.0, 1.5, 2.0, 2.5, 3.0, 3.5, 4.0, 4.5, 5.0, 6.0, 7, 8, 10, 12, 18, 24, 30, 36, and 48 hours after starting the infusion.

During the baclofen tablet administration treatment period, samples were drawn at 0 hour (predose) and at 0.25, 0.5, 0.75, 1.0, 1.5, 2.0 3.0, 3.5, 4.0, 5.0, 6.0, 7, 8, 10, 12, 18, 24, 30, 36, and 48 hours after oral administration.

Predose blood samples obtained from backup subjects who were randomized into the study may have exceeded the predose collection window.

Blood samples collected up to and including 12 hours post dose within ±2 minutes of scheduled time, and blood samples collected after 12 hours post dose within ±2 hours of scheduled time, were not considered deviations.

### Disposition of Subjects

A total of 34 subjects participated in the study; 32 of these subjects completed all study periods. Two subjects discontinued from the study early as described below. Subject X (19-year old, male) withdrew consent on Day 2 after receiving Treatment D (baclofen tablet 20 mg) in Period 1 and was not included in the PK analysis. Subject Y (29-year old, female) withdrew consent on Day 3 after receiving Treatment B (baclofen IV 16 mg infused over 180 minutes) in Period 1 and was not included in the PK analysis.

### Results

No clinical efficacy evaluation was conducted for this study. A summary of pharmacokinetic analyses was performed for use as a surrogate for efficacy and is detailed as below.

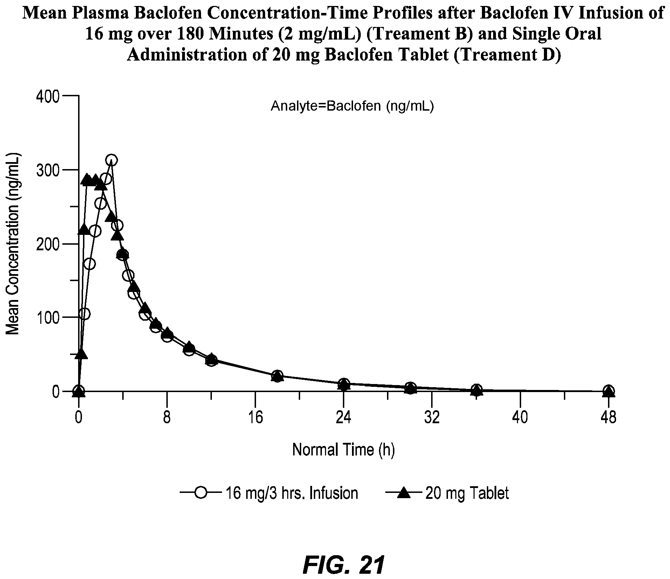

Mean concentration-time profile for the proposed dosing regimen is shown in Fig. 21.

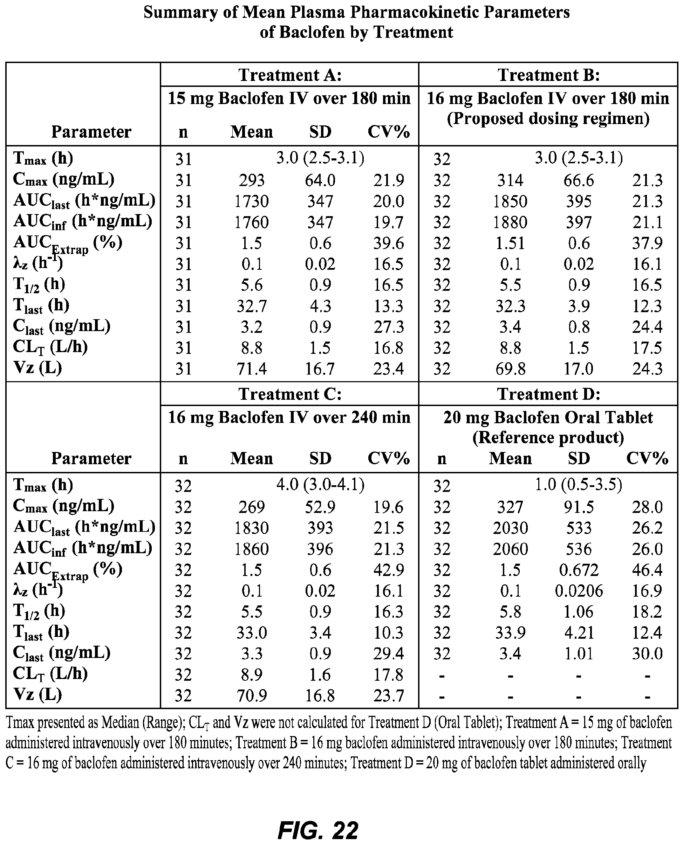

Mean pharmacokinetic parameters for baclofen are summarized in Fig. 22.

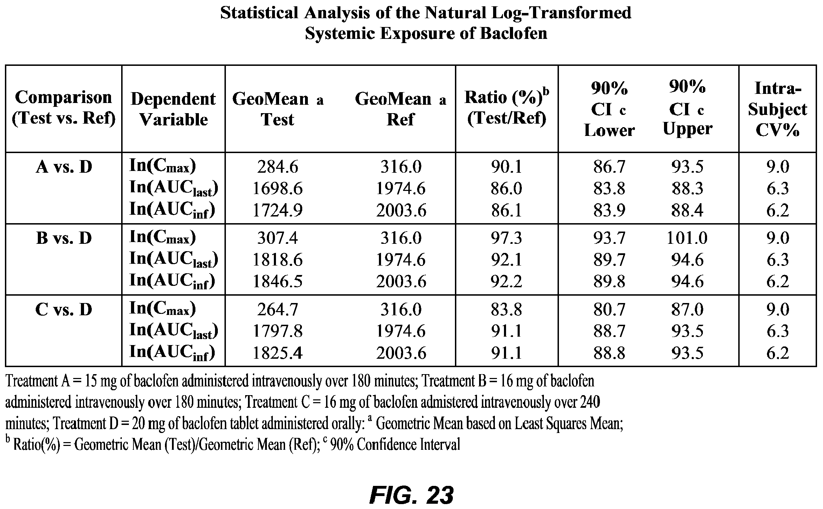

Results of the ANOVA for the log-transformed exposure parameters Cₘₐₓ, AUCₗₐₛₜ, and AUC_{inf} and the statistical analysis providing the 90% confidence interval for bioequivalence are presented in Fig. 23.

Median Tₘₐₓ occurred approximately 2.0 to 3.0 hours earlier for the oral tablet (1.0 h) compared to the IV infusions. Median Tₘₐₓ values were similar across IV infusions, ranging from 3.0-4.0 hours. Mean T_{1/2} were similar for IV infusions and the oral tablet; ranging from 5.5 h to 5.8 h.

Mean total systemic clearance (CL_{T}) and volume of distribution (Vz) values were similar across IV infusions. Mean CL_{T} ranged from 8.8 L/h to 8.9 L/h; mean Vz ranged from 69.8 L to 71.4 L.

All three baclofen IV infusions (15 mg of baclofen administered intravenously over 180 minutes; 16 mg of baclofen administered intravenously over 180 minutes; and 16 mg of baclofen administered intravenously over 240 minutes) were bioequivalent to the 20 mg baclofen oral tablet. Therefore, the study was concluded after Part 1.

Further, since the 16 mg baclofen administered intravenously over 180 minutes had the geometric mean ratios (90% confidence interval) vs. Treatment D (20 mg oral tablet) of 97.3% (93.7 - 101.0%) for Cₘₐₓ, 92.1% (89.7 - 94.6%) for AUCiast, and 92.2% (89.8 - 94.6%) for AUC_{inf} and demonstrated consistently a ratio of > 90% for every critical parameter, 16 mg baclofen administered over 180 minutes was chosen as the dosing regimen for registration.

Based on demonstrated bioequivalence, a conversion factor of 0.8 to the oral regimen administered over 180 minutes provides bioequivalent substitution for an oral dose of baclofen.

Although several embodiments have been described above with a certain degree of particularity, those skilled in the art could make numerous alterations to the disclosed embodiments without departing from the spirit of the present disclosure. It is intended that all matter contained in the above description or shown in the accompanying drawings shall be interpreted as illustrative only and not limiting. Changes in detail or structure may be made without departing from the present teachings. The foregoing description and following claims are intended to cover all such modifications and variations.

Any patent, publication, or other disclosure material, in whole or in part, that is said to be incorporated by reference herein is incorporated herein only to the extent that the incorporated materials does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

## Claims

1. Intravenous baclofen infusion for use as a medicament in a method of administering baclofen to a subject comprising:
administering an intravenous baclofen infusion comprising about 75% to about 85% of a therapeutically effective amount of oral baclofen over a time period of about 180 to about 240 minutes.

2. Intravenous baclofen infusion of claim 1, further comprising a sterile solution of baclofen at a concentration of about 2mg/mL.

3. Intravenous baclofen infusion of any of the preceding claims comprising about 80% of the therapeutically effective amount of oral baclofen.

4. Intravenous baclofen infusion of any of the preceding claims, wherein the therapeutically effective amount of oral baclofen is about 20 mg.

5. Intravenous baclofen infusion of any of the preceding claims, wherein the time period is about 180 minutes.

6. Intravenous baclofen infusion of any of the preceding claims, wherein the intravenous baclofen infusion is bioequivalent to the therapeutically effective amount of oral baclofen based on systemic exposure as defined by AUC.

7. Intravenous baclofen infusion of any of the preceding claims, wherein the administered intravenous baclofen infusion is bioequivalent to the therapeutically effective amount of oral baclofen based on maximum plasma concentration (Cₘₐₓ).

8. Intravenous baclofen infusion of any of the preceding claims , wherein administering the intravenous baclofen infusion further comprises administering about 16 mg baclofen over a time period of about 180 minutes.

9. Intravenous baclofen infusion of any of claims 1 to 7, wherein administering the intravenous baclofen infusion further comprises administering about 15 mg baclofen over a time period of about 180 minutes.

10. Intravenous baclofen infusion of any of claims 1 to 7, wherein administering the intravenous baclofen infusion further comprises administering about 16 mg baclofen over a time period of about 240 minutes.

11. Intravenous baclofen infusion of any of the preceding claims, wherein the method of administering baclofen to a subject comprises administering an intravenous baclofen infusion comprising about 80% of a therapeutically effective amount of oral baclofen over a time period of about 180 minutes.

12. Intravenous baclofen infusion of claim 11 , wherein administering the intravenous baclofen infusion comprising about 80% of the therapeutically effective amount of oral baclofen further comprises administering about 16 mg baclofen.

13. Intravenous baclofen infusion of any of the preceding claims, wherein the method of administering baclofen to a subject comprises:
administering the intravenous fluid to the subject over a specified time period to achieve a defined plasma baclofen delivery profile;
wherein the defined plasma baclofen delivery profile comprises a maximum plasma baclofen concentration of about 250 to about 350 ng/mL.

14. Intravenous baclofen infusion of claim13, wherein the specified time period is about 180 to about 240 minutes.

15. Intravenous baclofen infusion of any of the preceding claims, wherein the intravenous fluid comprises about 15 to about 16 mg baclofen.
